## Europäisches Patentamt
## European Patent Office
## Office européen des brevets

(19)

(11) Numéro de publication : **0 069 686**

**B1**

(12) **FASCICULE DE BREVET EUROPÉEN**

(45) Date de publication du fascicule du brevet :
22.01.86

(51) Int. Cl.⁴ : **A 61 M 5/315, A 61 J 1/00**

(21) Numéro de dépôt : **82420086.9**

(22) Date de dépôt : **29.06.82**

(54) Dispositif permettant le mélange à terme de deux ou plusieurs composants.

(30) Priorité : **29.06.81 FR 8113193**

(43) Date de publication de la demande :
**12.01.83 Bulletin 83/02**

(45) Mention de la délivrance du brevet :
**22.01.86 Bulletin 86/04**

(84) Etats contractants désignés :
**AT BE CH DE GB IT LI LU NL SE**

(56) Documents cités :
**DE-A- 2 316 209**
**US-A- 2 841 145**
**US-A- 3 052 239**
**US-A- 3 489 147**
**US-A- 3 680 558**

(73) Titulaire : **Lontrade, Jean**
**124, Avenue Thermale**
**Chamalières Puy de Dôme (FR)**

(72) Inventeur : **Dupont, Philippe**
**3 rue Colbert**
**Clermont-Ferrand Puy de Dôme (FR)**
Inventeur : **Lontrade, Jean**
**124, Avenue Thermale**
**Chamalieres Puy de Dôme (FR)**

(74) Mandataire : **Maureau, Bernard**
**Cabinet GERMAIN & MAUREAU Le Britannia - Tour C**
**20, Boulevard Eugène Déruelle**
**F-69003 Lyon (FR)**

Jouve, 18, rue St-Denis, 75001 Paris, France

EP 0 069 686 B1

## Description

La présente invention concerne un dispositif permettant le mélange à terme de deux ou plusieurs composants contenus dans le dispositif au moment de l'emploi.

On sait que, notamment dans le domaine des préparations médicamenteuses, il est souvent impératif, que ce soit en raison de l'instabilité du produit final ou de la nécessité de conservation stérile des produits de base, de ne procéder au mélange des constituants essentiels de la préparation qu'au moment de son utilisation.

De nombreux dispositifs ont été déjà proposés pour effectuer ce mélange ; ces dispositifs sont le plus souvent constitués par deux chambres séparées par une membrane, et c'est la perforation ou la rupture de cette membrane qui permet d'assurer au moment de l'emploi le mélange des constituants élémentaires.

On connaît d'autre part des seringues conçues dans le même but ; il s'agit le plus souvent de dispositifs composés de deux corps de seringue constituant respectivement les deux chambres renfermant les constituants élémentaires du mélange, le piston ne jouant alors que le rôle, qui lui est classiquement dévolu, de pousser le liquide contenu dans la chambre dans laquelle il est logé.

Le document DE-A-2 316 209, décrit un dispositif ayant une chambre de contenance fixe qui présente une seule ouverture et est séparée de la chambre par l'intermédiaire d'un joint souple dont le positionnement permet de rompre provisoirement l'étanchéité afin d'obtenir un mélange, et de la rétablir à la demande.

Des descriptions de seringues du type ci-dessus sont données dans les documents US-A-2 841 145, et US-A-3 680 558. Dans le dispositif décrit dans le document US-A-3 680 558 la mise en communication des deux corps de seringue est assurée par l'intermédiaire d'un joint constitué de deux moyens d'obturation comprenant des orifices susceptibles d'être amenés en coïncidence. Dans le dispositif décrit dans le document US-A-2 841 145, une des chambres est constituée par un corps dont une des extrémités est ouverte et comporte sur sa face extérieure un filetage, tandis que le joint souple a la forme d'une coupelle cylindrique dont la paroi de l'alésage présente un filetage apte à coopérer avec le filetage externe du corps pour permettre leur vissage mutuel, au moins un orifice traversant de part en part le fond de la coupelle de telle sorte que, par vissage du corps dans le joint, on obtient la fermeture étanche de la chambre par suite de l'engagement mutuel du fond de la coupelle et de l'extrémité filetée du corps et qu'inversement, par dévissage du corps dans le joint, on provoque l'ouverture de la chambre mettant ainsi en communication les chambres.

Dans le document US-A-3 489 147, c'est la rupture d'une membrane étanche séparant les deux corps de seringue qui assure la mise en communication des deux chambres. Il s'agit donc là de dispositifs complexes dans leur conception et coûteux dans leur réalisation.

Un dispositif simplifié a été proposé dans le document US-A-3 052 239 dans lequel c'est le piston qui constitue l'une des chambres, l'autre chambre étant constituée par le corps de la seringue ; il s'agit également d'un dispositif complexe puisque ce piston comporte trois ouvertures, absolument nécessaires pour la mise en service de la seringue que décrit ce brevet : une ouverture supérieure (fermée par un bouchon amovible) destinée au remplissage du piston, une ouverture dans l'embout pour permettre la sortie du liquide et une ouverture de « ventilation » destinée à rompre le vide établi dans le corps de la seringue lors de l'introduction, dans cette dernière, de l'autre élément du mélange.

La présente invention s'est donnée pour but de proposer un nouveau dispositif permettant le mélange à terme de deux composants, d'une réalisation plus simple et plus économique que les dispositifs mentionnés ci-avant et qui permette, en outre, de rétablir, une fois le mélange effectué, l'étanchéité dans la chambre contenant ce mélange.

C'est ainsi qu'elle a pour objet un dispositif permettant le mélange à terme de deux ou plusieurs composants, ce dispositif comportant deux chambres distinctes contenant les composants séparées de manière étanche par l'intermédiaire d'un joint souple présentant la forme d'une coupelle cylindrique dont le positionnement par rapport au corps d'une première de ces chambres dont le volume est ainsi variable permet de rompre provisoirement l'étanchéité entre celles-ci afin d'obtenir un mélange des composants et de la rétablir à la demande, une extrémité du corps de ladite première chambre comportant une ouverture en bout et, sur sa surface cylindrique extérieure, un filetage apte à coopérer avec un filetage complémentaire ménagé sur la paroi intérieure de la coupelle cylindrique du joint souple, au moins un orifice de communication avec la deuxième chambre traversant de part en part le fond de la coupelle, ce dispositif étant caractérisé en ce que le corps de la première chambre de volume variable, ne comporte qu'une seule ouverture, en ce que cette ouverture se présente sous l'aspect d'une embouchure en forme de tronc de cône s'évasant vers l'extérieur en regard de l'autre chambre et en ce que le fond de la coupelle cylindrique formant le joint souple présente du côté de la première chambre une saillie tronconique évasée en direction du fond coaxiale à l'axe de ladite coupelle et directement complémentaire du profil tronconique femelle de ladite embouchure, plusieurs orifices traversant de part en part le fond de la coupelle et étant disposés à la périphérie de la base de la saillie tronconique de telle sorte que par vissage du corps de la première chambre dans le joint, on obtient la fermeture étanche de la première chambre par suite de l'engagement mutuel de la saillie tronconique et de la paroi tronconique délimitant l'embouchure et qu'inversement, par dévissage du corps de la première chambre dans le joint, on provoque l'ouverture de celle-ci, la mettant ainsi en communication avec la deuxième chambre.

On dispose ainsi d'un dispositif très simple (puisque la chambre étanche ne comporte qu'une seule ouverture), n'exigeant pas l'application de vide pour réaliser l'étanchéité et d'emploi très intéressant puisqu'il est à tout moment possible de rétablir l'étanchéité entre les deux chambres une fois le mélange réalisé.

La présente invention sera mieux comprise d'ailleurs et ses avantages ressortiront bien de la description qui suit de trois modes d'exécution du dispositif selon l'invention en référence au dessin schématique annexé dans lequel :

Les figures 1 à 4 représentent un mode de réalisation du dispositif selon l'invention appliqués à une seringue ;

Figure 1 étant une vue éclatée en coupe d'une seringue et d'un joint selon l'invention ;

Figure 2 étant une vue en coupe de la seringue montée selon figure 1 ;

Figure 3 étant une vue en coupe de la seringue selon figure 1 en position de mélange des constituants ;

Figure 4 étant une vue en coupe selon 4-4 de figure 2.

Les figures 5 et 6 représentent un mode de réalisation du dispositif selon l'invention, appliqué aux flacons munis d'en embout souple, et dans le cas représenté d'une tête de collyre.

Sur les figures, (2) désigne la première chambre contenant le composant (3), (4) la deuxième chambre contenant le composant (5) et (6) le joint souple selon l'invention assurant la séparation et le maintien en position relative des deux chambres (2) et (4) et équipé de moyens lui permettant en une étape ultérieure de rompre provisoirement l'étanchéité dans l'une des chambres (2) tout en libérant dans l'autre chambre (4) le composant qu'elle contient, puis de rétablir cette étanchéité. Selon l'invention, la chambre (2), de volume variable présente une seule ouverture (10).

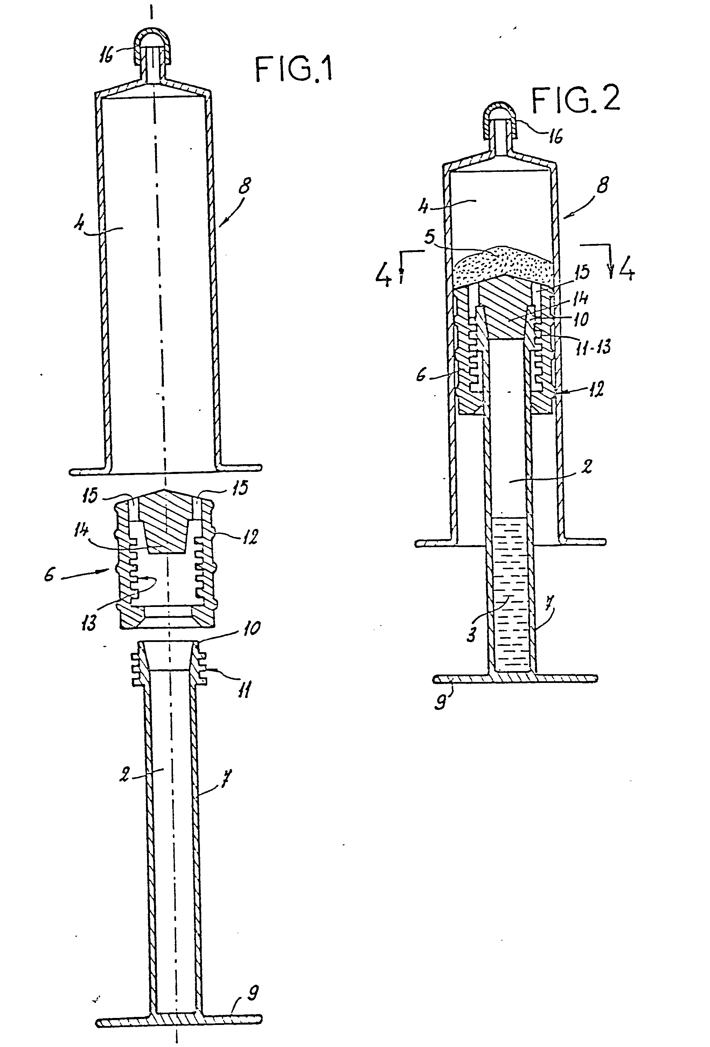

Dans le mode de réalisation représenté aux figures 1 à 4, c'est le piston (7) de la seringue (8) qui constitue la chambre (2) dans laquelle on introduit le constituant (3), en l'espèce l'élément liquide, le corps de la seringue constituant la deuxième chambre (4) contenant le deuxième constituant (5) du futur mélange.

La seringue (8) représentée au dessin, présente la forme générale d'un cylindre ovalisé ; elle peut également présenter une section circulaire. Dans ce cas la face interne de la seringue et/ou la face externe du joint présentent des systèmes de blocage antirotation (non représentés).

Le piston (7) est équipé à l'une de ses extrémités, comme il est connu en soi, d'un dispositif de préhension (9), des ailettes dans le cas représenté au dessin, mais il peut également s'agir d'un anneau ou de tout dispositif similaire. L'embout (10) du corps du piston (7) est ouvert selon une embouchure en forme de tronc de cône s'évasant vers l'extérieur ; il comporte, sur sa surface cylindrique extérieure, un filetage (11).

Le bouchage de cet orifice est opéré par un joint souple (16) de la forme d'une coupelle cylindrique dont la paroi de l'alésage présente un filetage (13) apte à coopérer avec le filetage (11) du corps (7) pour permettre leur solidarisation.

Le fond de la coupelle présente une saillie tronconique (14) évasée en direction du fond, coaxiale à l'axe de ladite coupelle et directement complémentaire du profil tronconique femelle de l'ouverture (10) du corps (7) tandis que sont prévus des orifices (15) traversant de part en part le fond de la coupelle et disposés à la périphérie de la base de la saillie tronconique (14) de telle sorte que par vissage du corps (7) dans le joint (6), on obtient la fermeture étanche de la chambre (2) par suite de l'engagement mutuel de la saillie tronconique (14) et de la paroi tronconique délimitant l'ouverture (10) et qu'inversement, par dévissage du corps (7) dans le joint (6) on provoque l'ouverture de la chambre (2) mettant ainsi en communication les chambres (2) et (4).

En une première étape, on introduit le premier composant (3), en l'espèce l'élément liquide du futur mélange, par l'extrémité ouverte (10) du piston (7) ; on visse ensuite le piston (7) dans le joint souple (6).

L'emmanchement des deux parties tronconiques complémentaires (10) et (14) assure un bouchage étanche.

L'autre constituant (5) du futur mélange (sous forme liquide ou pulvérulente) est alors introduit dans le corps de la seringue (8) dont l'extrémité est bouchée par un capuchon amovible (16).

Puis l'ensemble piston (7), joint souple (6) est introduit dans le corps de la seringue (8).

De façon générale, l'ordre et les méthodes de remplissage ne sont pas l'objet de ce brevet et peuvent être modifiés selon les besoins spécifiques des produits mis en œuvre ou des machines dont disposent les conditionneurs.

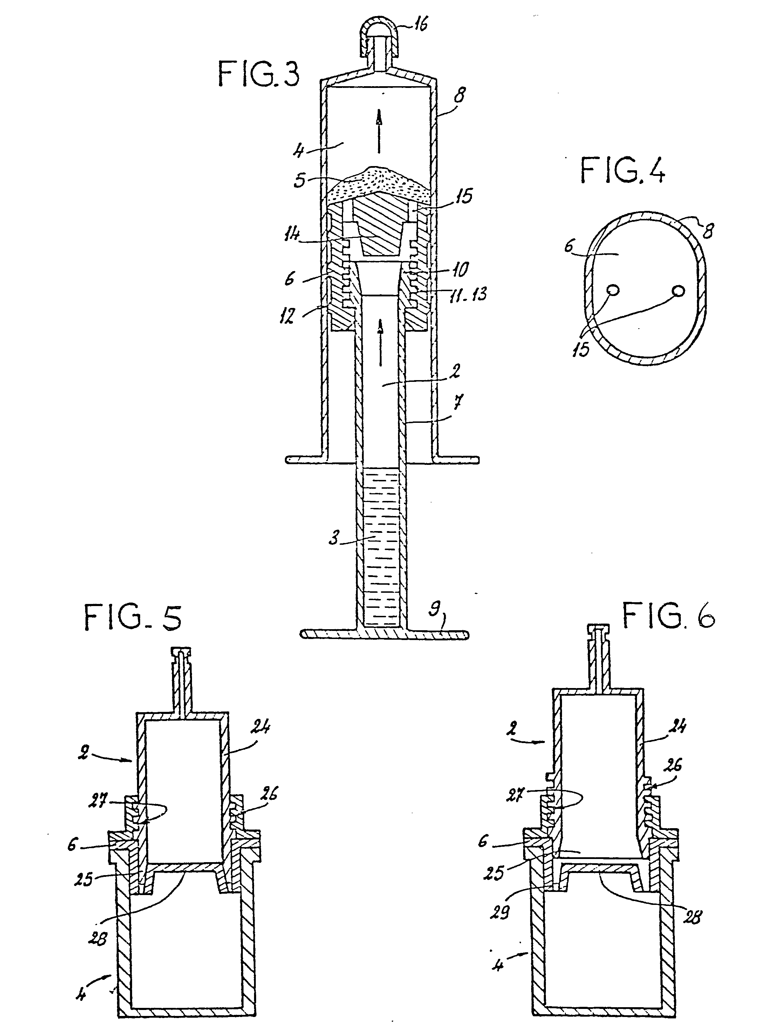

Lors de l'emploi, l'utilisateur tenant d'une main la seringue (8) dévisse de l'autre main le piston (7), libérant ainsi l'assemblage des deux parties tronconiques complémentaires (10) et (14) (figure 3). On tire ensuite le piston (7), le liquide (3) est alors aspiré du piston vers la partie inférieure (4) de la seringue en passant par les orifices (15) et vient se mélanger au constituant (5) ; on revisse l'ensemble, on enlève le capuchon amovible (16) et on adapte l'aiguille pour procéder à l'injection.

Dans le cas où le composant (5) est prélevé par l'aspiration provoquée par le recul de l'ensemble piston-joint (dans la position étanche) à l'intérieur de la seringue (8) pour être ensuite mélangé avec les composants contenus dans le piston (7), le mélange peut se faire de façon quantitative ou non grâce à l'évaluation des volumes dans les deux chambres selon l'application retenue.

Le dispositif représenté aux figures 5 et 6 peut être adapté aux flacons munis d'un embout souple.

Ce mode de réalisation se rapproche de celui représenté aux figures 1 à 3. La chambre (2) est constituée par un embout souple (24) dont l'extrémité (25) est ouverte, présente la forme d'un tronc de cône s'évasant vers l'extérieur comporte sur sa face extérieure un filetage (26) et le joint souple (6) a la forme d'une coupelle cylindrique dont la paroi de l'alésage présente un filetage (27) apte à coopérer avec le filetage (26) de l'embout souple (24) pour permettre leur vissage mutuel, le fond de la coupelle présentant une saillie tronconique (28) évasée en direction du fond coaxiale à l'axe de ladite coupelle et directement complémentaire du profil tronconique femelle de l'extrémité (25) de l'embout souple (24), tandis que sont prévus des orifices (29) traversant de part en part le fond de la coupelle et disposés à la périphérie de la base de la saillie tronconique (28).

Au moment de l'emploi, il suffit à l'utilisateur de dévisser l'embout souple (24) libérant ainsi l'assemblage des deux parties tronconiques complémentaires (25) et (28) ; l'élément contenu dans la tête (24) va se mélanger à celui renfermé dans le flacon constituant la chambre (4).

Comme il va de soi et comme il ressort de ce qui précède, la présente invention ne se limite pas aux deux formes d'exécution décrites ci-dessus à titre d'exemples ; elle en embrasse, au contraire, toutes les variantes de réalisation, quelles que soient notamment la nature du ou des éléments constituant le joint souple et la nature des éléments constituant les chambres (2) et (4), la nature des composants contenus ou non dans lesdites chambres ; elle s'applique notamment au cas des mélanges devant être faits avec des composants prélevés extemporanément.

**Revendications**

1. Dispositif permettant le mélange à terme de deux ou plusieurs composants, ce dispositif comportant deux chambres distinctes (2, 4) contenant les composants séparées de manière étanche pour ceux-ci par l'intermédiaire d'un joint souple (6) présentant la forme d'une coupelle cylindrique dont le positionnement par rapport au corps d'une première (2) de ces chambres dont le volume est ainsi variable permet de rompre provisoirement l'étanchéité entre celles-ci afin d'obtenir un mélange des composants et de la rétablir à la demande, une extrémité du corps de cette première chambre (2) comportant en bout, une ouverture et, sur sa surface cylindrique extérieure, un filetage (11, 26) apte à coopérer avec un filetage complémentaire (13, 27) ménagé sur la paroi intérieure de la coupelle cylindrique du joint, au moins un orifice de communication avec la deuxième chambre (4) traversant de part en part le fond de la coupelle, caractérisé en ce que le corps de la première chambre (2) de volume variable ne comporte qu'une seule ouverture, en ce que cette ouverture se présente sous l'aspect d'une embouchure (10, 25) en forme de tronc de cône s'évasant vers l'extérieur en regard de l'autre chambre et en ce que le fond de la coupelle cylindrique formant le joint souple présente du côté de la première chambre une saillie tronconique (14, 18) évasée en direction du fond, coaxiale à l'axe de ladite coupelle et directement complémentaire du profil tronconique femelle de ladite embouchure (10, 25), plusieurs orifices (15, 29) traversant de part en part le fond de la coupelle et étant disposés à la périphérie de la base de la saillie tronconique (14, 28) de telle sorte que par vissage du corps (7, 24) de la première chambre (2) dans le joint (6) on obtient la fermeture étanche de celle-ci par suite de l'engagement mutuel de la saillie tronconique (14, 28) et de la paroi tronconique délimitant l'embouchure (10, 25) et qu'inversement, par dévissage du corps (7, 24) de la première chambre (2) dans le joint (6) on provoque l'ouverture de celle-ci, la mettant ainsi en communication avec la deuxième chambre (4).

2. Dispositif selon la revendication 1, caractérisé en ce que le corps (7) de la première chambre (2) est constitué par le piston d'une seringue.

3. Dispositif selon la revendication 1, caractérisé en ce que le corps (24) de la première chambre (2) est constitué par un embout souple.

4. Dispositif selon la revendication 2, caractérisé en ce que le corps de la seringue est un cylindre ovalisé.

5. Dispositif selon la revendication 2, caractérisé en ce que le corps de la seringue est un cylindre de révolution et en ce que l'alésage dudit cylindre et/ou la surface extérieure du joint monté coulissant dans celui-ci présente des systèmes de blocage antirotation.

**Claims**

1. An appliance permitting two or more components to be mixed after a period, this appliance comprising two separate chambers (2, 4) containing the components, separated in a manner impervious to them by the interposition of a flexible joint (6) having the shape of a cylindrical obturator, the position of which relatively to a first chamber (2), the volume of which is in this manner variable, enables the seal between the two chambers to be temporarily broken in order to effect a mixture of the components and to be re-setablished as required, one extremity of the body of this first chamber (2) comprising an end opening and on its external surface a screw thread (11, 26) suitable to work in conjunction with a complementary thread (13, 27) provided on the internal wall of the cylindrical obturator of the joint, at least one orifice communicating with the second chamber (4) and passing right through the base of the obturator, characterized in that the body of the first chamber (2) of variable volume only possesses a single opening ; in that this opening appears in the form of a mouthpiece shaped like a truncated cone widening out towards the exterior in relation to the other chamber ; and in that the base of the cylindrical obturator forming the flexible joint possesses on the side facing the first chamber a truncated conical projection (14, 18) opening out towards the base, coaxial with the axis of the said obturator and directly complementary to the female truncated conical profile of the said mouthpiece (10, 25), several orifices (15, 29) passing right through the base of the obturator and being located at the periphery of the truncated conical projection (14, 28) in such a way that by screwing the body (7, 24) of the first chamber (2) into the joint (6) sealed closure of the latter is obtained resulting from the mutual engagement of the truncated conical projection (14, 28) and the truncated conical wall (10, 25) forming the boundary of the mouthpiece, and that inversely, by unscrewing the body (7, 24) of the first chamber (2) in the joint (6) the latter is caused to open, thus placing the first chamber in communication with the second chamber (4).

2. An appliance according to Claim 1, characterized in that the body (7) of the first chamber (2) is formed by the piston of a syringe.

3. An appliance according to Claim 1, characterized in that the body (24) of the first chamber (2) is formed by a flexible ferrule.

4. An appliance according to Claim 2, characterized in that the body of the syringe is an ovalized cylinder.

5. An appliance according to Claim 2, characterized in that the body of the syringe is a cylinder of revolution and in that the bore of the said cylinder and/or the external surface of the mounted joint sliding in it possesses systems blocking them against rotation.

## Patentansprüche

1. Einrichtung zum Mischen von zwei oder mehr Komponenten, umfassend zwei die Komponenten enthaltende, getrennte Kammern (2, 4), welche in einer für die Komponenten dichten Weise voneinander durch ein Dichtsitzorgan (6) getrennt sind, welches die Form eines zylindrischen Tiegels aufweist, dessen Position im Bezug auf das Gehäuse einer ersten (2) dieser Kammern, deren Volumen auf diese Weise variabel ist, es ermöglicht, zeitweise die Abdichtung zwischen diesen Kammern aufzuheben, um ein Vermischen der Komponenten zu erreichen, und die Abdichtung bei Bedarf wieder herzustellen, wobei ein Ende des Gehäuses dieser ersten Kammer (2) an der Stirnseite eine Öffnung und an seiner zylindrischen Außenfläche ein Gewinde (11, 26) aufweist, welches mit einem komplementären, an der Innenwand des zylindrischen Tiegels des Dichtorgans (6) ausgebildeten Gegengewinde zusammenwirkt und wobei wenigstens eine Durchgangsöffnung für eine Verbindung mit der zweiten Kammer (4) vorgesehen ist, welche den Boden des Tiegels von einer Seite zur andern durchdringt, dadurch gekennzeichnet, daß das Gehäuse der ersten Kammer (2) mit variablem Volumen nur eine Öffnung aufweist, daß diese Öffnung als Mündung (10, 25) mit der Form eines Kegelstumpfes ausgebildet ist, welcher sich bezüglich der anderen Kammer nach außen erweitert, und daß der Boden des zylindrischen Tiegels, welcher das Dichtsitz-Organ (6) bildet, auf seiner der ersten Kammer zugewandten Seite einen kegelstumpfförmigen Vorsprung (14, 28) bildet, welcher sich in Richtung zu diesem Boden hin erweitert und welcher koaxial zur Achse des Tiegels und direkt komplementär zum aufnehmenden, kegelstumpfförmigen Profil der Mündung (10, 25) ausgebildet ist, daß mehrere Durchgangsöffnungen (15, 29) den Boden von einer Seite zur anderen durchsetzen, wobei die Durchgangsöffnungen an der Peripherie der Basis des kegelstumpfförmigen Vorsprunges (14, 28) derart angeordnet sind, daß durch Einschrauben des Gehäuses (7, 24) der ersten Kammer (2) in das Dichtsitz-Organ (6) eine dichte Verschließung derselben erreicht wird als Folge der gegenseitigen Anlage des kegelstumpfförmigen Vorsprunges (14, 28) und der kegelstumpfförmigen Wand, welche die Mündung (10, 25) begrenzt, und daß umgekehrt durch Losschrauben des Gehäuses (7, 24) der ersten Kammer (2) im Dichtsitz-Organ (6) eine Öffnung derselben erreicht wird, wodurch eine Verbindung mit der zweiten Kammer (4) hergestellt wird.

2. Einrichtung nach Anspruch 1, dadurch gekennzeichnet, daß das Gehäuse (7) der ersten Kammer (2) durch den Kolben einer Injektionsspritze gebildet wird.

3. Einrichtung nach Anspruch 1, dadurch gekennzeichnet, daß das Gehäuse (24) der ersten Kammer (2) durch eine elastische Kappe gebildet wird.

4. Einrichtung nach Anspruch 2, dadurch gekennzeichnet, daß das Gehäuse der Injektionsspritze ein Zylinder mit etwa ovalem Querschnitt ist.

5. Einrichtung nach Anspruch 2, dadurch gekennzeichnet, daß das Gehäuse der Injektionsspritze ein rotationssymmetrischer Zylinder ist und daß der Innenraum des des Zylinders und/oder die Außenfläche des gleitend in diesem gelagerten Dichtorgans Einrichtungen für eine Drehsperrung aufweisen.

FIG.1

FIG.2

FIG.3

FIG.4

FIG. 5

FIG. 6